# EUROPÄISCHE PATENTSCHRIFT

(11) **EP 2 621 456 B1**
(45) Veröffentlichungstag und Bekanntmachung des Hinweises auf die Patenterteilung: **06.12.2017**
(21) Anmeldenummer: 11761584.9
(22) Anmeldetag: 27.09.2011
(51) Int. Cl.: A61K 8/04, A61K 8/11, A61K 8/81, A61K 8/92, A61Q 19/10, A61Q 5/02, A61K 8/46, C11D 1/02, C11D 1/88, C11D 1/94, C11D 3/37, C11D 17/00, A61K 8/02

(54) **Verfahren zur Herstellung eines kosmetischen Reiningungsmittels**
Process for the preparation of a cosmetic cleanser
Procédé pour la préparation d'un produit cosmétique nettoyant

(30) Priorität: 29.09.2010 DE 102010041596
(43) Veröffentlichungstag der Anmeldung: 07.08.2013
(73) Patentinhaber: Henkel AG & Co. KGaA, 40589 Düsseldorf (DE)
(72) Erfinder: SARTINGEN, Kirsten, 41379 Brüggen (DE); PETERSOHN, Dirk, 50996 Köln (DE)
(86) Internationale Anmeldenummer: PCT/EP2011/066729
(87) Internationale Veröffentlichungsnummer: WO 2012/041831

(56) Entgegenhaltungen:
- EP-A1- 1 700 619
- EP-A2- 2 036 590
- EP-A2- 2 070 511
- WO-A1-03/074021
- WO-A1-2008/110740
- US-A1- 2004 023 820

## Beschreibung

Die Erfindung liegt auf dem Gebiet der kosmetischen Mittel und betrifft ein Herstellungsverfahren für ein fließfähiges Reinigungsmittel, das neben Tensiden und einem speziellen Polymer eine dispergierte feste Komponente enthält.

Kosmetische Reinigungsmittel, die feste Teilchen enthalten, sind aus dem Stand der Technik bekannt.
Von vielen Verbrauchern werden solche Produkte aufgrund ihres ansprechenden optischen Erscheinungsbildes bevorzugt.
Sie weisen den Vorteil auf, dass beispielsweise hydrolyse-, licht- und/oder oxidationsempfindliche Wirkstoffe und/oder in dem tensidhaltigen Medium instabile oder unlösliche Komponenten in die festen Teilchen eingearbeitet werden können. Die Teilchen werden erst bei der Anwendung des Reinigungsmittels durch Reibung zerstört und die Wirkstoffe freigesetzt, wodurch sie ihre volle Wirkung auf der Anwendungsoberfläche entfalten können.
Die Herstellung und die Stabilisierung solcher Produkte sind jedoch problematisch, denn feste Teilchen neigen in fließfähigen Zubereitungen dazu nach oben steigen, wodurch das Produkt sein ästhetisches Erscheinungsbild verliert.
Ein weiteres Problem ist die Viskositätseinstellung und -erhaltung solcher Mittel, denn auch eine Viskositätsänderung kann zu unerwünschtem Aufsteigen der festen Teilchen und/oder Gasblasen führen.
In der Patentanmeldung EP 1210402 werden transparente Reinigungszusammensetzungen offenbart, die neben Tensiden und kationischen Polymeren teilweise neutralisierte anionische Acrylat-Copolymere sowie unlösliche Komponenten wie Kugeln, teilchenförmige Stoffe, wasserunlösliche Flüssigkeiten und/oder Glasblasen enthalten.
Um die erforderliche Viskosität für die Stabilisierung der unlöslichen Komponenten zu erreichen, werden die Reinigungszusammensetzungen auf pH-Werte von 5,5 bis 7 eingestellt.
Neuere Haut- und/oder Haarreinigungsmittel weisen pH-Werte im aciden Bereich auf, um den natürlichen Säureschutzmantel der Haut/der Kopfhaut zu unterstützen bzw. während der Reinigung nicht oder möglichst wenig aus seinem Gleichgewicht zu bringen.

Zusammensetzungen, die auf anionischen Acrylat-Polymeren als Stabilisierungsmittel für unlösliche Komponenten beruhen, sind für den aciden pH-Bereich ungeeignet, denn sie verlieren im aciden Bereich (bei pH-Werten unterhalb von 7) ihre Verdickungswirkung (und damit ihre Stabilisierungswirkung).
Ein weiterer Nachteil bei der Verwendung anionischer Acrylat-Copolymere zur Stabilisierung fester Komponenten in fließfähigen Zusammensetzungen besteht in der langwierigen Vorquellung der Acrylat-Copolymere, gegebenenfalls bei erhöhten Temperaturen.
Ziel der vorliegenden Erfindung war es, fließfähige kosmetische Reinigungszusammensetzungen herzustellen, die dispergierte, feste Komponenten enthalten, welche auch bei pH-Werten im aciden Bereich (unterhalb von 6) in den Reinigungszusammensetzungen stabilisiert werden können.
Ein weiteres Ziel der vorliegenden Erfindung war es, die Herstellung der fließfähigen Zusammensetzungen weniger zeit- und kostenaufwändig sowie aus ökologischen Gesichtspunkten energiesparender zu gestalten.
Vorgeschlagen werden daher fließfähige kosmetische Reinigungsmittel, die neben bestimmten Tensidklassen sowie festen Komponenten mit Aminogruppen substituierte (Meth)Acrylat-Copolymere enthalten.

Gegenstand der vorliegenden Erfindung wird in Anspruch 1 definiert. Unter "fließfähig" sind Zusammensetzungen zu verstehen, die bei 20°C bevorzugt eine Viskosität im Bereich von 1,000 bis 20,000 mPas, mehr bevorzugt von 5,000 bis 11,000 mPas, besonders bevorzugt im Bereich von 6,000 bis 10,000 mPas und insbesondere im Bereich von 6,500 bis 9,500 mPas aufweisen (gemessen mit einem Haake Rotationsviskosimeter VT550; 20°C; Meßeinrichtung MV; Spindel MV II; 8 UPM).

Die Reinigungsmittel sind bevorzugt wässrig und/oder wässrig-alkoholisch. Unter "wässrig" wird verstanden, dass die erfindungsgemäßen Reinigungsmittel bevorzugt mindestens 50 Gew.-%, mehr bevorzugt mindestens 55 Gew.-%, besonders bevorzugt mindestens 60 Gew.-% und insbesondere mindestens 65 Gew.-% Wasser enthalten.

Fakultativ können die Reinigungsmittel zusätzlich maximal 20 Gew.-%, bevorzugt maximal 15 Gew.-%, mehr bevorzugt maximal 10 Gew.-% und insbesondere maximal 7,5 Gew.-% mindestens eines Alkohols enthalten.
Geeignete Alkohole sind Ethanol, 1-Propanol, 2-Propanol, Isopropanol, 1,2-Propylenglycol, 1-Butanol, 2-Butanol, Isobutanol, 1,2-Butylenglycol, 1-Pentanol, 2-Pentanol, 1,2-Pentandiol, 1-Haxanol, 2-Hexanol, 1,2-Hexandiol, Glycerin, Sorbitol sowie aromatische Alkohole wie beispielsweise Phenoxyethanol.
Besonders bevorzugt sind Ethanol, 1,2-Propylenglycol, Sorbitol und Glycerin. Insbesondere bevorzugt sind Ethanol und/oder Glycerin.
In einer bevorzugten Ausführungsform der Erfindung weisen die fließfähigen Reinigungsmittel einen pH-Wert im Bereich von 3,5 bis 6, mehr bevorzugt von 4 bis 5,5 und insbesondere von 4,5 bis 5 auf.
In einer weiteren bevorzugten Ausführungsform der Erfindung sind die fließfähigen Reinigungsmittel transparent. Diese Ausführungsform wird von Verbrauchern bevorzugt, da sie ästhetisch ansprechender ist und den Vorteil aufweist, dass die transparente Phase und die darin dispergierte Phase (enthaltend gegebenenfalls Wirk- und Pflegestoffe) besser visualisiert werden können.
Unter "transparenten" fließfähigen Reinigungszusammensetzungen werden Reinigungszusammensetzungen verstanden, deren nicht-dispergierte (transparente) Phase bevorzugt einen NTU-Wert (Nephelomteric Turbidity Unit) von maximal 100, mehr bevorzugt von maximal 75, besonders bevorzugt von maximal 50 und insbesondere von maximal 30 aufweist.

Die Reinigungsmittel enthalten mindestens ein Tensid, welches aus anionischen und/oder amphoteren/zwitterionischen Tensiden ausgewählt sein kann.
Geeignete anionische Tenside werden den erfindungsgemäßen Reinigungszusammensetzungen - bezogen auf deren Gesamtgewicht - bevorzugt in Mengen von 0,5 bis 25 Gew.-%, mehr bevorzugt von 1 bis 20 Gew.-%, besonders bevorzugt von 2 bis 17,5 Gew.-% und insbesondere von 3 bis 15 Gew.-% zugegeben.
Zu den geeigneten anionischen Tensiden, die in den erfindungsgemäßen Reinigungsmitteln eingesetzt werden können, zählen:
- geradkettige oder verzweigte, gesättigte oder ein- bzw. mehrfach ungesättigte Alkylsulfonate mit 8 bis 24 C-Atomen,
- lineare Alpha-Olefinsulfonate mit 8 bis 24 C-Atomen,
- Alkylsulfate und Alkylpolyglykolethersulfate der Formel R-O-(CH₂-CH₂O)ₙ-O-SO₃X, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali- oder Erdalkalimetall oder für Triethanolamin steht,
- Sulfonate ungesättigter Fettsäuren mit 8 bis 24 C-Atomen und 1 bis 6 Doppelbindungen.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten.
Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Geeignete amphotere/zwitterionische Tenside können den erfindungsgemäßen Reinigungsmitteln - bezogen auf deren Gesamtgewicht - bevorzugt in Mengen von 0,1 bis 20 Gew.-%, mehr bevorzugt von 0,25 bis 15 Gew.-%, besonders bevorzugt von 0,5 bis 12,5 und insbesondere von 1 bis 10 Gew.-% zugegeben werden.

Geeignete amphotere/zwitterionische Tenside können ausgewählt sein aus Verbindungen der folgenden Formeln (i) bis (v), in denen der Rest R jeweils für einen geradkettigen oder verzweigten, gesättigten oder ein- bzw. mehrfach ungesättigten Alkyl- oder Alkenlyrest mit 8 bis 24 Kohlenstoffatomen steht,

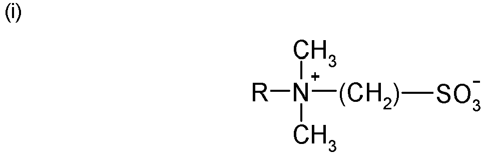

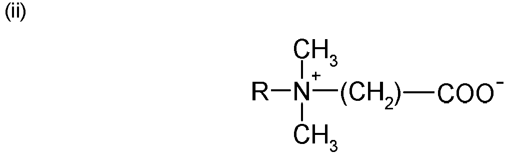

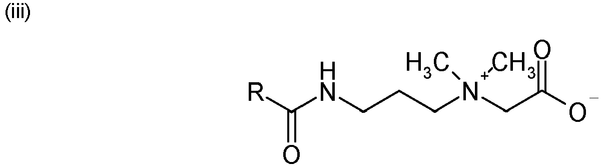

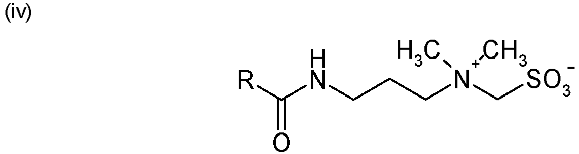

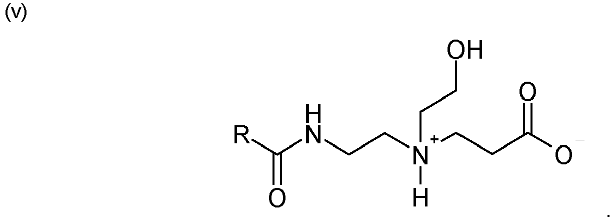

Zu den insbesondere geeigneten amphoteren/zwitterionischen Tensiden zählen die unter der INCI-Bezeichnung bekannten Tenside Cocamidopropylbetain und Disodium Cocoamphodiacetate.

In einer besonders bevorzugten Ausführungsform der Erfindung enthalten die Reinigungsmittel anionische und amphotere Tenside aus den zuvor genannten Gruppen. Besonders milde Zubereitungen können erhalten werden, wenn die Reinigungszusammensetzungen mindestens ein anionisches Tensid und mindestens ein amphoteres/zwitterionisches Tensid in einem Verhältnis von 5 : 1 bis 1 : 3, bevorzugt von 4,5 : 1 bis 1 : 1,5 und insbesondere von 4 : 1 bis 1 : 1 enthalten.

Geeignete, mit Aminogruppen substituierte (Meth)acrylat-Copolymere b), sind bevorzugt Copolymere eines oder mehrerer einfacher Ester der Acryl- oder der Methacrylsäure.
Besonders bevorzugte Copolymere b) sind vernetzt, vorzugsweise mit Ethylenglycol Dimethacrylat. Ganz besonders bevorzugte Copolymere b) sind vernetzte (Meth)acrylat-Copolymere, die C₁₋₄-(Meth)acrylat-Momomere, Monomere, welche Aminogruppierungen enthalten, sowie gegebenenfalls hydrophobe und/oder hydrophob modifizierte Monomere enthalten.
Insbesondere bevorzugt sind die unter der INCI-Bezeichnung "Polyacrylate-1 Crosspolymer" bekannten Polymere.

Das Copolymer b) kann den Reinigungszusammensetzungen - bezogen auf deren Gesamtgewicht - bevorzugt in Mengen von 0,01 bis 20 Gew.-%, mehr bevorzugt von 0,05 bis 15 Gew.-%, besonders bevorzugt von 0,1 bis 10 Gew.-% und insbesondere von 0,5 bis 5 Gew.-% zugegeben werden.

Alternativ kann das Copolymer b) den Reinigungszusammensetzungen bereits als Emulsion zugegeben werden. Das hat den verfahrensökonomischen Vorteil, dass eine Vorquellung des Polymeren in Wasser nicht mehr notwenig ist.

Als geeignete Emulsion, enthaltend ein zuvor beschriebenes Copolymer b), kann beispielsweise das Handelsprodukt "Carbopol Aqua CC" in den erfindungsgemäßen Reinigungszusammensetzungen eingesetzt werden.
"Carbopol Aqua CC" kann in den erfindungsgemäßen Reinigungszusammensetzungen - bezogen auf deren Gesamtgewicht - bevorzugt in einer Menge von 1 bis 30 Gew.-%, mehr bevorzugt von 2 bis 20 Gew.-%, besonders bevorzugt von 3 bis 15 Gew.-% und insbesondere in einer Menge von 4 bis 12 Gew.-% eingesetzt werden.

Als geeignete feste in dem Reinigungsmittel dispergierte Komponente c), kommen beispielsweise in Betracht:
- teilchenförmige Stoffe wie beispielsweise die Abrasivstoffe Aluminiumoxid, Kunststoffpartikel, Kieselsäure, Polysiloxan, Weizenkleie und/oder Mandelkleie,
- Pigmente,
- hohle Perlen,
- mit Flüssigkeit(en) und/oder Feststoff(en) gefüllte Perlen.
Die dispergierte Komponente c) kann den erfindungsgemäßen Reinigungsmitteln - bezogen auf deren Gesamtgewicht - bevorzugt in Mengen von 0,01 bis 15 Gew.-%, mehr bevorzugt von 0,025 bis 10 Gew.-%, besonders bevorzugt von 0,05 bis 7,5 Gew.-% und insbesondere von 0,1 bis 5 Gew.-% hinzugefügt werden.
Die dispergierte Komponente c) weist in dem Reinigungsmittel bevorzugt eine Partikel- bzw. Blasengröße von 10 bis 3500 µm, bevorzugt von 50 bis 3000 µm, mehr bevorzugt von 100 bis 2500 µm und insbesondere von 250 bis 2000 µm auf.
In einer bevorzugten Ausführungsform werden den erfindungsgemäßen Reinigungsmitteln als Komponente(n) c) Perlen hinzugefügt, die bevorzugt mit Flüssigkeit(en) und/oder Feststoff(en) gefüllt sind.
Innerhalb dieser Ausführungsform ist es bevorzugt, wenn die Perlen aus einer in dem Reinigungsmittel unlöslichen Hülle bestehen und mit Wirkstoffen gefüllt sind, die bei der Anwendung des Reinigungsmittels freigesetzt werden.
Die Freisetzung der Wirkstoffe aus den Perlen erfolgt bevorzugt, indem das Mittel mit den Händen auf der Anwendungsoberfläche verteilt wird. Dabei wird mit den Händen Druck erzeugt, wodurch die Hülle der Perlen zerstört wird, und die Wirkstoffe austreten können.
Als in dem Reinigungsmittel unlösliche Hülle der Perlen kommt bevorzugt eine Hülle aus Polymeren, Gelatine und/oder pflanzlichen Gummen in Frage.

Besonders bevorzugte Perlen enthalten als Hülle eine Mischung aus (Fisch-)Gelatine und/oder pflanzlichen Gummen wie Gummi arabicum, Gummi Benzoe, Gummi Guajaci, Gummi Mastix, Gummi Styrax und Gummi Traganth.
Insbesondere bevorzugt sind Perlen mit einer Hülle, die aus einer Mischung aus (Fisch-)Gelatine und Gummi arabicum besteht.

Innerhalb dieser Ausführungsform ist es weiterhin bevorzugt, wenn die Perlen Wirkstoffe enthalten, die in dem Reinigungsmittel instabil sind und/oder in freier Form in dem Reinigungsmittel durch zusätzliche Inhaltsstoffe stabilisiert werden müssten.
Geeignete Wirkstoffe, die im Inneren der Perlen enthalten sein können, sind beispielsweise:
- tierische, pflanzliche und/oder synthetische Öle und/oder Wachse,
- mit Metalloxiden beschichtetes Mica,
- Konservierungsmittel,
- Abrasivstoffe,
- UV-Filter und/oder
- Vitamine.

Bevorzugte Öle, die in den Perlen enthalten sein können, sind beispielsweise Silikone, sowie natürliche Öle und/oder Wachse synthetischen, pflanzlichen und tierischen Ursprungs.

Geeignete Silikone enthalten mindestens ein Vertreter aus der Gruppe siliciumorganischer Verbindungen, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl in Frage.
Besonders bevorzugt sind Jojobaöl, Avocadoöl, Aprikosenkernöl, Hagebuttenkernöl, Mandelöl, Olivenöl, Pfirsichkernöl, Shea-Butter, Sonnenblumenöl und Traubenkernöl.

Geeignete Wachse können pflanzlichen, mineralischen, synthetischen oder tierischen Ursprungs sein.
Natürliche oder pflanzliche Wachse sind bevorzugt Carnaubawachs, Candelillawachs, Japanwachs, Zuckerrohrwachs und Jojobaöl.
Tierische Wachse sind bevorzugt Bienenwachs, Wollwachs, Bürzeldrüselfett und Walrat. Natürliche und synthetische Mineralwachse sind bevorzugt Paraffine, Ceresin und Ozokerit sowie Polyethylenglycolwachse.

Unter geeigneten Vitaminen sind bevorzugt Vitamine, Provitamine und Vitaminvorstufen sowie deren Derivate zu verstehen:
Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
Vitamin B: zur Vitamin B-Gruppe oder zu dem Vitamin B-Komplex gehören u. a.
   - Vitamin B₁ (Thiamin)
   - Vitamin B₂ (Riboflavin)
   - Vitamin B₃. Unter dieser Bezeichnung werden häufig die Verbindungen Nicotinsäure und Nicotinsäureamid (Niacinamid) geführt.
   - Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
   - Vitamin B₆ (Pyridoxin sowie Pyridoxamin und Pyridoxal).
Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol).
Vitamin F: unter dem Begriff "Vitamin F" werden üblicherweise essentielle Fettsäuren, insbesondere Linolsäure, Linolensäure und Arachidonsäure, verstanden.
Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.
Die Perlen können bevorzugt Vitamine, Provitamine und Vitaminvorstufen aus den Gruppen A, B, E und H enthalten.

Geeignete UV-Filter sind alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.
Weitere geeignete UV-Filter enthalten eine kationische Gruppe, insbesondere eine quartäre Ammoniumgruppe.
Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyldimethylaminobenzamidopropyl-dimethylammoniumtosylat (Escalol® HP 610).

Innerhalb dieser Ausführungsform ist es besonders bevorzugt, wenn die Perlen eine Hülle aus (Fisch-)Gelatine und/oder pflanzlichen Gummen, und im Inneren mindestens ein pflanzliches Öl enthalten.

Insbesondere bevorzugt sind Perlen, die eine Hülle aus (Fisch-)Gelatine und Gummi arabicum, und im Inneren pflanzliche Öle wie Jojobaöl, Avocadoöl, Aprikosenkernöl, Hagebuttenkernöl, Mandelöl, Olivenöl, Pfirsichkernöl, Shea-Butter, Sonnenblumenöl und Traubenkernöl enthalten.
Die Perlen können zur besseren Visualisierung mit einem physiologisch verträglichen Farbstoff angefärbt sein.
Geeignete Handelsprodukte, die als Komponente c) in den erfindungsgemäßen Reinigungsmitteln eingesetzt werden können, sind beispielsweise die von der Firma ISP erhältlichen "Captivates HC", wie ISP Captivates® HC 4915, ISP Captivates® HC 4855, ISP Captivates® HC 4848, ISP Captivates® HC 4787, ISP Captivates® HC 4769, ISP Captivates® HC 4023 und/oder ISP Captivates® HC 1843.

Die Einsatzmenge des kationischen Polymeren in den Reinigungsmitteln beträgt - bezogen auf deren Gesamtgewicht - bevorzugt 0,01 bis 10 Gew.-%, mehr bevorzugt 0,025 bis 7,5 Gew.-%, besonders bevorzugt 0,05 bis 5 Gew.-% und insbesondere 0,1 bis 2,5 Gew.-%.
Geeignete kationische Polymere sind beispielsweise:
- quaternisierte Cellulose-Derivate, wie sie unter den Bezeichnungen Celquat® und Polymer JR® im Handel erhältlich sind,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.
Weitere geeignete kationischen Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind.
Besonders bevorzugte kationische Polymere, die in den erfindungsgemäßen Reinigungsmitteln eingesetzt werden können, sind quaternisierte Cellulosepolymere, kationische Guarderivate und/oder kationische Polymere auf Acrylsäure(derivat)basis, die insbesondere ausgewählt sind aus den unter den INCI-Bezeichnungen bekannten Polymeren Polyquaternium-6, Polyquaternium-7, Polyquaternium-10 und/oder Polyquaternium-67.

Die Reinigungsmittel eignen sich für die Anwendung auf der Haut und/oder den Haaren und können bevorzugt als Duschgel, Duschbad, Schaumbad, Gesichtspeeling, flüssige Handwaschseife und/oder Haarshampoo konfektioniert werden.
Innerhalb dieser Konfektionierungsformen können die Reinigungsmittel neben den zuvor beschriebenen Komponenten weitere Komponenten enthalten, die den Mitteln vorteilhafte Eigenschaften verleihen.

Zu den weiteren fakultativen Komponenten zählen beispielsweise
- weitere, von a) verschiedene Tenside,
- Pflanzenextrakte und/oder
- Feuchthaltemittel.

Geeignete, von a) verschiedene Tenside sind bevorzugt nichtionische Tenside und/oder kationische Tenside.

Zu den bevorzugten nichtionischen Tensiden zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside.

Für den Fall, dass ein weiteres nichtionisches Tensid in den Reinigungszusammensetzungen eingesetzt wird, sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, bevorzugt.

Die fakultativen nichtionischen Tenside können in den erfindungsgemäßen Reinigungsmitteln - bezogen auf deren Gesamtgewicht - bevorzugt in einer Menge von 0,05 bis 10 Gew.-%, mehr bevorzugt von 0,1 bis 7,5 Gew.-%, besonders bevorzugt von 0,2 bis 5 Gew.-% und insbesondere in einer Menge von 0,25 bis 3 Gew.-% eingesetzt werden.

Geeignete kationische Tenside sind beispielsweise quartäre Ammoniumverbindungen, Esterquats und/oder Amidoamine.
Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyldimethylamin dar.

Die fakultativen kationischen Tenside können in den erfindungsgemäßen Reinigungsmitteln bevorzugt in einer Menge von 0,05 bis 10 Gew.-%, bezogen auf das gesamte Mittel, eingesetzt werden. Mengen von 0,1 bis 5 Gew.-% sind besonders bevorzugt.

Unter geeigneten Pflanzenextrakten sind Extrakte zu verstehen, die aus allen Teilen einer Pflanze hergestellt werden können.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktio nsmittelgemisch.

Geeignete Feuchthaltemittel bzw. Penetrationshilfsstoffe und/ oder Quellmittel, die den erfindungsgemäßen Reinigungsmitteln zugesetzt werden können, sind beispielsweise Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.
Besonders geeignet ist Glycerin.

Die Feuchthaltemittel können in den erfindungsgemäßen Zusammensetzungen - bezogen auf deren Gesamtgewicht - in Mengen von 0,01 bis 10 Gew.-%, bevorzugt in Mengen von 0,05 bis 5 Gew.-% und insbesondere in Mengen von 0,1 bis 3 Gew.-% eingesetzt werden.

Weitere Wirk-, Hilfs- und Zusatzstoffe, die in den erfindungsgemäßen Reinigungsmitteln eingesetzt werden können, sind beispielsweise:
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise α- und β-Hydroxycarbonsäuren,
- Wirkstoffe wie Allantoin und Bisabolol,
- Cholesterin,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konsistenzgeber wie Zuckerester, Polyolester oder Polyolalkylether,
- Konservierungsmittel, wie beispielsweise Natriumbenzoat,
- Viskositätsreglerwie Salze (NaCl).
Neben einem ästhetischen Erscheinungsbild haben die fließfähigen kosmetischen Reinigungsmittel den Vorteil, dass sie, bedingt durch die Anwesenheit des Copolymeren b), im aciden Bereich formuliert werden können, ohne dass die Verdickungs- und Stabilisierungswirkung des Polymeren b) verloren geht. Die dispergierte(n) Komponente(n) c) wird (werden) auch in dem aciden Reinigungsmittel in der Schwebe gehalten.
Ein weiteres Ziel der vorliegenden Erfindung war es, die Herstellung der fließfähigen Zusammensetzungen weniger zeit- und kostenaufwändig sowie aus ökologischen Gesichtspunkten energiesparender zu gestalten.
In früheren Herstellungsverfahren von Reinigungsmitteln, die dispergierte Komponenten und "Acrylat-Copolyere" als Verdickungs- und/oder Stabilisierungsmittel enthalten, musste zunächst die Struktur der Acrylat-Copolyere aufgebaut werden, bevor die übrigen Inhaltsstoffe hinzugefügt wurden.
Anderenfalls konnten keine klaren Zusammensetzungen hergestellt werden, und in der Zusammensetzung dispergierte Komponenten stiegen nach oben.
Eine weiterer Nachteil des früheren Verfahrens war (insbesondere dann, wenn feste Komponenten in die Zusammensetzung eingearbeitet werden sollten), dass die Herstellung unter Vakuum erfolgen musste, da anderenfalls auch durch Rühren in die Zusammensetzungen eingeschleppte Luftblasen darin stabilisiert wurden.
Diese Nachteile machten die Herstellung zeitaufwendig (durch die Vorquellung des Carbopols) und kostenaufwendig (bedingt durch den erhöhten Energieaufwand bei der Erzeugung eines Vakuums).
Diese Nachteile konnten beseitigt werden, indem ein neuartiges, mit Aminogruppen substituiertes (Meth)acrylat-Copolymer in neuartiger Art und Weise in ein kosmetisches Reinigungsmittel eingearbeitet wurde.

Bevorzugt wird in dem Schritt (ii) des erfindungsgemäßen Verfahrens das Handelsprodukt "Carbopol Aqua CC" eingesetzt.
Bevorzugt ist weiterhin, dass in dem Schritt (ii) eine 1 : 1 Mischung der Emulsion "Carbopol Aqua CC" mit destilliertem Wasser hergestellt wird.
Das erfindungsgemäße Verfahren weist den Vorteil auf, dass das Verdickungsmittel (Carbopol) nicht wie bisher üblich vorgelegt, vorgequollen und neutralisiert werden muss, bevor die übrigen Inhaltsstoffe hinzugefügt werden.
Stattdessen wird es als Premix mit Wasser (Schritt (ii)) am Ende des Herstellungsprozesses, d.h. nach der Vermischung aller Komponenten (Schritt (i)) hinzugefügt.
Für den Fall, dass feste dispergierte Komponenten in das Reinigungsmittel eingearbeitet werden, ist es erst dann und nur für einen kurzen Zeitraum notwenig, zur Entfernung von Luftblasen ein Vakuum anzulegen.
Nach der anschließenden Neutralisation und Viskositätseinstellung erfolgt erst der Strukturaufbau des Carbopols, wodurch die dispergierte(n) Komponente(n) in dem Reinigungsmittel in der Schwebe gehalten werden.
Es können klare Rezepturen erhalten werden.
Ein weiterer Vorteil ist, dass mit dem erfindungsgemäßen Verfahren die Viskositätseinstellung mit NaCl möglich ist.

### Beispiel:

**Duschgel:**

| | Menge in Gew.-% |
|---|---|
| Natriumlaurethsulfat (2 EO) | 9 |
| Cocamidopropylbetain | 3 |
| Glycerin | 1 |
| Polyquaternium-7 | 0,1 |
| PEG-7 Glyceryl Cocoate | 0,8 |
| Carbopol®¹ Aqua CC | 7 |
| ISP Captivates®² HC 4769 | 0,5 |
| NaCl, Citronensäure, Parfum, Konservierungsmittel | q.s. |
| Wasser | ad 100 |

| | |
|---|---|
| Es wurden die folgenden Handelsprodukte eingesetzt: ¹ INCI-Bezeichnung: Polyacrylate-1 Crosspolymer; (20% Feststoffe in Wasser); Lubrizol, ² INCI-Bezeichnung: Aqua (Water), Helianthus Annuus (Sunflower) Seed Oil, Gelatin, Acacia Senegal Gum, Vitis Vinifera (Grape) Seed Oil, Mica, Cl 77891 (Titanium Dioxide), Cl 77491 (Iron Oxides), Silica, Xanthan Gum, Tin Oxide; ISP | |

Das Duschgel der Tabelle wurde hergestellt, indem die Tenside, Glycerin, das kationische Polymer, PEG-7 Glyceryl Cocoate, das Parfum und die Konservierungsmittel vermischt wurden.
Carbopol Aqua CC wurde mit Wasser im Verhältnis 1 : 1 vermischt und zu der Tensidmischung gegeben. Anschließend wurde ein Vakuum angelegt, um die Luftblasen aus dem Duschgel zu entfernen.
Dann wurde mit Citronensäure ein pH-Wert von etwa 4,5 bis 5 eingestellt und mit NaCl die gewünschte Viskosität eingestellt.

Bei dem Duschgel handelt es sich um eine transparente Rezeptur, in der goldgelbe Perlen schweben, die mit Sonnenblumenöl und Traubenkernöl gefüllt sind.
Das Duschgel ist unter üblichen Lagerungsbedingungen stabil und zeigt weder Trübungserscheinungen noch ein Aufsteigen der Perlen.

## Patentansprüche

1. Verfahren zur Herstellung eines fließfähigen kosmetischen Reinigungsmittels, enthaltend feste Komponenten, **dadurch gekennzeichnet, dass**
(i) in einem ersten Schritt
- ein oder mehrere Tensid(e), ausgewählt aus anionischen und/oder amphoteren/zwitterionischen Tensiden,
- feste Komponenten,
- Wasser sowie
- gegebenenfalls weitere fakultative Inhaltsstoffe miteinander vermischt werden,
(ii) in einem zweiten Schritt ein mit Aminogruppen substituiertes, vernetztes (Meth)Acrylat-Copolymer a) oder ein Handelsprodukt, enthaltend ein mit Aminogruppen substituiertes vernetztes (Meth)Acrylat-Copolymer b), mit Wasser vermischt und zu der Mischung (i) gegeben wird, und
(iii) die Mischung aus (i) und (ii) in einem dritten Schritt neutralisiert und anschließend - bevorzugt mit Natriumchlorid - verdickt wird,
wobei das kosmetische Reinigungsmittel einen pH-Wert im Bereich von 3,5 bis 6, bevorzugt von 4 bis 5,5 und insbesondere von 4,5 bis 5 aufweist.

2. Verfahren nach Anspruch 1, **dadurch gekennzeichnet, dass** das Reinigungsmittel - bezogen auf sein Gesamtgewicht -
a1) 0,5 bis 25 Gew.-%, bevorzugt 1 bis 20 Gew.-%, mehr bevorzugt 2 bis 17,5 Gew.-% und insbesondere 3 bis 15 Gew.-% mindestens eines anionischen Tensids, ausgewählt aus geradkettigen und/oder verzweigten Alkylsulfaten und/odersulfonaten mit einem Alkylrest von 8 bis 18, insbesondere von 10 bis 16 C-Atomen und mit 0 bis 10, bevorzugt mit 1 bis 6 und insbesondere mit 2 bis 4, Ethylenoxideinheiten, und/oder
a2) 0,1 bis 20 Gew.-%, bevorzugt 0,25 bis 15 Gew.-%, mehr bevorzugt 0,5 bis 12,5 Gew.-% und insbesondere 1 bis 10 Gew.-% mindestens eines amphoteren/zwitterionischen Tensids, ausgewählt aus den unter der INCI-Bezeichnung Cocamidopropyl Betain und/oder den unter der INCI-Bezeichnung Disodium Cocoamphodiacetate bekannten Verbindungen, enthält.

3. Verfahren nach einem der Ansprüche 1 oder 2, **dadurch gekennzeichnet, dass** das Copolymer b) C₁₋₄-(Meth)acrylat-Momomere, Monomere, welche Aminogruppierungen enthalten, sowie gegebenenfalls hydrophobe und/oder hydrophob modifizierte Monomere enthält, und vorzugsweise mit Ethylenglycol Dimethacrylat vernetzt ist.

4. Verfahren nach einem der Ansprüche 1 bis 3, **dadurch gekennzeichnet, dass** das Reinigungsmittel - bezogen auf sein Gesamtgewicht - 0,01 bis 20 Gew.-%, bevorzugt 0,05 bis 15 Gew.-%, mehr bevorzugt 0,1 bis 10 Gew.-% und insbesondere 0,5 bis 5 Gew.-% des Copolymeren b) enthält.

5. Verfahren nach einem der Ansprüche 1 bis 4, **dadurch gekennzeichnet, dass** das Reinigungsmittel - bezogen auf sein Gesamtgewicht - 0,01 bis 15 Gew.-%, bevorzugt 0,025 bis 10 Gew.-%, mehr bevorzugt 0,05 bis 7,5 Gew.-% und insbesondere 0,05 bis 5 Gew.-% mindestens einer festen dispergierten Komponente c) enthält, die ausgewählt ist aus teilchenförmigen Stoffen, Pigmenten, hohlen Perlen und/oder mit Flüssigkeit(en) und/oder Feststoff(en) gefüllten Perlen.

6. Verfahren nach Anspruch 5, **dadurch gekennzeichnet, dass** die Komponente c) in dem Reinigungsmittel eine Partikelgröße von 10 bis 3500 µm, bevorzugt von 50 bis 3000 µm, mehr bevorzugt von 100 bis 2500 µm und insbesondere von 250 bis 2000 µm aufweist.

7. Verfahren nach einem der Ansprüche 5 oder 6, **dadurch gekennzeichnet, dass** das Reinigungsmittel als wasserunlösliche Komponente c) Perlen enthält, die eine Hülle aus Gelatine und/oder pflanzlichen Gummen aufweisen, und im Inneren mindestens ein pflanzliches ÖI enthalten.

8. Verfahren nach einem der Ansprüche 1 bis 7, **dadurch gekennzeichnet, dass** das Reinigungsmittel - bezogen auf sein Gesamtgewicht - 0,01 bis 10 Gew.-%, bevorzugt 0,025 bis 7,5 Gew.-%, mehr bevorzugt 0,05 bis 5 und insbesondere 0,1 bis 2,5 Gew.-% mindestens eines- von b) verschiedenen- kationischen Polymeren enthält.

## Claims

1. A method for preparing a flowable cosmetic cleansing agent, containing solid components, **characterized in that**
(i) in a first step,
- one or more surfactant(s), selected from anionic and/or amphoteric/zwitterionic surfactants,
- solid components,
- water, and
- possibly further optional ingredients, are mixed together,
(ii) in a second step, a cross-linked (meth)acrylate copolymer a) that is substituted with amino groups, or a commercial product containing a cross-linked (meth)acrylate copolymer b) that is substituted with amino groups, is mixed with water and added to the mixture (i), and
(iii) the mixture from (i) and (ii) is neutralized in a third step and subsequently thickened, preferably using sodium chloride,
the cosmetic cleansing agent having a pH in the range of from 3.5 to 6, preferably from 4 to 5.5, and in particular from 4.5 to 5.

2. The method according to claim 1, **characterized in that** the cleansing agent contains, based on the total weight thereof,
a1) from 0.5 to 25 wt.%, preferably from 1 to 20 wt.%, more preferably from 2 to 17.5 wt.% and in particular from 3 to 15 wt.%, of at least one anionic surfactant selected from straight-chain and/or branched alkyl sulfates and/or sulfonates having an alkyl functional group having from 8 to 18, in particular from 10 to 16, C-atoms and having from 0 to 10, preferably having from 1 to 6, and in particular having from 2 to 4, ethylene oxide units, and/or
a2) from 0.1 to 20 wt.%, preferably from 0.25 to 15 wt.%, more preferably from 0.5 to 12.5 wt.%, and in particular from 1 to 10 wt.%, of at least one amphoteric/zwitterionic surfactant selected from the compounds known by the INCI name Cocamidopropyl Betain and/or by the INCI name Disodium Cocoamphodiacetate.

3. The method according to either claim 1 or claim 2, **characterized in that** copolymer b) contains C₁₋₄ (meth)acrylate monomers, monomers that contain amino groupings, and optionally hydrophobic and/or hydrophobically modified monomers, and is preferably cross-linked with ethylene glycol dimethacrylate.

4. The method according to one of claims 1 to 3, **characterized in that** the cleansing agent contains, based on the total weight thereof, from 0.01 to 20 wt.%, preferably from 0.05 to 15 wt.%, more preferably from 0.1 to 10 wt.%, and in particular from 0.5 to 5 wt.% of copolymer b).

5. The method according to one of claims 1 to 4, **characterized in that** the cleansing agent contains, based on the total weight thereof, from 0.01 to 15 wt.%, preferably from 0.025 to 10 wt.%, more preferably from 0.05 to 7.5 wt.%, and in particular from 0.05 to 5 wt.%, of at least one solid dispersed component c) that is selected from particulate substances, pigments, hollow pearls and/or pearls filled with liquid(s) and/or solid(s).

6. The method according to claim 5, **characterized in that** component c) has a particle size, in the cleansing agent, of from 10 to 3500 µm, preferably from 50 to 3000 µm, more preferably from 100 to 2500 µm, and in particular from 250 to 2000 µm.

7. The method according to either claim 5 or claim 6, **characterized in that** the cleansing agent contains pearls as water-insoluble component c), which pearls have a casing made of gelatin and/or vegetable gummas, and contain at least one vegetable oil in the inside thereof.

8. The method according to one of claims 1 to 7, **characterized in that** the cleansing agent contains, based on the total weight thereof, from 0.01 to 10 wt.%, preferably from 0.025 to 7.5 wt.%, more preferably from 0.05 to 5 wt.%, and in particular from 0.1 to 2.5 wt.%, of at least one cationic polymer that is different from b).

## Revendications

1. Procédé de fabrication d'un produit nettoyant cosmétique fluide, contenant des composants solides, **caractérisé en ce que** :
(i) dans une première étape
- au moins un tensioactif choisi parmi des tensioactifs anioniques et/ou amphotères/zwitterioniques,
- des composants solides,
- de l'eau, ainsi qu'éventuellement
- d'autres ingrédients facultatifs mélangés entre eux,
(ii) dans une deuxième étape, un copolymère (méth)acrylate réticulé substitué par des groupes amino a) ou un produit commercial contenant un copolymère (méth)acrylate réticulé substitué par des groupes amino b), est mélangé à de l'eau et est ajouté au mélange (i), et
(iii) dans une troisième étape, le mélange de (i) et (ii) est neutralisé puis épaissi - de préférence avec du chlorure de sodium,
où le produit nettoyant cosmétique présente un pH entre 3,5 et 6, de préférence entre 4 et 5,5 et en particulier entre 4,5 et 5.

2. Procédé selon la revendication 1, **caractérisé en ce que** le produit nettoyant contient-rapporté à son poids total :
a1) 0,5 à 25 % en poids, de préférence 1 à 20 % en poids, plus préférentiellement 2 à 17,5 % en poids et en particulier 3 à 15 % en poids d'au moins un tensioactif anionique choisi parmi des sulfates et/ou sulfonates d'alkyle linéaires et/ou ramifiés avec un résidu alkyle de 8 à 18, en particulier de 10 à 16 atomes de C et 0 à 10, de préférence 1 à 6 et en particulier 2 à 4 unités oxyde d'éthylène, et/ou
a2) 0,1 bis 20 % en poids, de préférence 0,25 à 15 % en poids, plus préférentiellement 0,5 à 12,5 % en poids et en particulier 1 à 10 % en poids d'au moins un tensioactif amphotère/zwitterionique choisi parmi les composés connus par les désignations INCl cocamidopropyl bétaïne et/ou cocoamphodiacétate disodique.

3. Procédé selon une des revendications 1 ou 2, **caractérisé en ce que** le copolymère b) contient des monomères C₁₋₄-(méth)acrytates, des monomères contenant des groupes amino, ainsi qu'éventuellement des monomères hydrophobes et/ou modifiés hydrophobes, et est préférentiellement réticulé par du diméthacrylate d'éthylène glycol.

4. Procédé selon une des revendications 1 à 3, **caractérisé en ce que** le produit nettoyant contient - rapporté à son poids total - 0,01 à 20 % en poids, de préférence 0,05 à 15 % en poids, plus préférentiellement 0,1 à 10 % en poids et en particulier 0,5 à 5 % en poids du copolymère b).

5. Procédé selon une des revendications 1 à 4, **caractérisé en ce que** le produit nettoyant contient - rapporté à son poids total - 0,01 à 15 % en poids, de préférence 0,025 à 10 % en poids, plus préférentiellement 0,05 à 7,5 % en poids et en particulier 0,05 à 5 % en poids d'au moins un composant solide dispersé c), qui est choisi parmi des substances particulaires, pigments, perles creuses et/ou perles remplies d'un ou plusieurs liquides et/ou solides.

6. Procédé selon la revendication 5, **caractérisé en ce que** les composants c) dans le produit nettoyant présentent une granulométrie de 10 à 3500 pm, de préférence 50 à 3000 pm, plus préférentiellement 100 à 2500 pm et en particulier 250 à 2000 µm.

7. Procédé selon une des revendications 5 ou 6, **caractérisé en ce que** le produit nettoyant contient comme composants insolubles dans l'eau c) des perles, qui présentent une coque de gélatine et/ou de gommes végétales, et à l'intérieur au moins une huile végétale.

8. Procédé selon une des revendications 1 à 7, **caractérisé en ce que** le produit nettoyant contient - rapporté à son poids total - 0,01 à 10 % en poids, de préférence 0,025 à 7,5 % en poids, plus préférentiellement 0,05 à 5 et en particulier 0,1 à 2,5 % en poids d'au moins un polymère cationique différent de b).
